# EUROPEAN PATENT APPLICATION

(11) **EP 0 528 076 A1**
(43) Date of publication of application: **24.02.1993**
(21) Application number: 91202116.9
(22) Date of filing: 20.08.1991
(51) Int. Cl.: A61K 31/235

(54) **Use of Mebeverine against non-specific inflammatory bowel disease**

(71) Applicant: DUPHAR INTERNATIONAL RESEARCH B.V, NL-1381 CP Weesp (NL)
(72) Inventor: Vantrappen,G, Weesp (NL)
(74) Representative: Breepoel, Peter M.

(57) **Abstract**

The present invention is concerned with medicaments effective against non-specific inflammatory bowel disease, such as as ulcerative colitis and Crohn's disease, containing mebeverine.

## Description

The present invention is concerned with the preparation of a medicament effective against non-specific inflammatory bowel disease, and with a medicament suitable for release of the active compound in the diseased area.

Non-specific inflammatory bowel disease comprises afflictions like ulcerative colitis and Crohn's disease.

Symptoms of these diseases are diarrhoea, rectal bleeding, abdominal pain, cramping, anorexia and weight loss.

Conventionally, these diseases are medicated with e.g. 5-ASA containing products (like sulfasalazine, mesalamine), alsalazine, azathioprine, corticosteroids (such as betamethasone, prednisolone, methylprednisolone), and the like.

Generally these medicaments show side-effects. Since sulfasalazine is metabolized to sulfapyridine and 5-aminosalicylic acid (mesalamine), its adverse effects are similar to those of sulfonamides and of salicylates. Common dose related side-effects of these sulfasalazine compounds include: nausea, headache, and anorexia. In higher doses accelerated hemolysis is often observed. Adverse reactions not related to the dose or serum sulfapyridine levels include: fever, rash, hepatic dysfunction, toxic epidermal necrolysis, agranulocytosis, thrombocytopenia, pancreatitis, pulmonary infiltrates and serum sickness type reactions.
This spectrum of reactions is typical of the sulfonamides in general. Finally, sulfasalazine has been shown to cause infertility in males. The recenty introduced mesalamines also produce unwanted effects including interstitial nephritis, nephrotic syndrome and pyuria. Adverse effects of corticosteriods are dependent on the preparation and the dose. In general side effects result in disturbances in electrolyte balance, sodium and water retention, with oedema and hypertension. Other metabolic effects include induction of osteoporosis, hyperglycemia, increased susceptibility to all kind of infections. Other adverse effects include amenorrhoea, hyperleideosis, mental and neurological disturbances, intracranial hypertension, acute pancreatitis and aseptic necrosis of bone. An increase in the coagulability of the blood may lead to thrombo-embolic complications.

Therefore, there is clearly a need for a medicament effective against inflammatory bowel disease, which shows less side-effects.

The present invention provides a medicament for effectively combating non-specific inflammatory bowel disease, which contains a therapeutically effective amount of mebeverine, preferably in a formulation suitable for release of the mebeverine in the diseased area.

Mebeverine is a well known anti-spasmodic agent, with the chemical name 3,4-dimethoxybenzoic acid 4-[ethyl-[2-(4-methoxyphenyl)-1-methylethyl]amino]butyl ester.

Formulations suitable for release of the mebeverine in the diseased area are e.g. formulations for rectal administration, or formulations for oral administration with a sustained or controled release at the diseased intestine (ileum and/or colon).

For use by rectal administration, the medicament may, appart from mebeverine, also contain one or more compounds effective in the enhancement of rectal compliance. Compounds suitable for this purpose are e.g. laudanum and loperamide.
Furthermore, the medicament may contain one or more other active component effective in enhancement of the therapeutic efficacy of the mebeverine, in particular 5-ASA containing products, corticosteroids, immunosuppressive agents and the like. The advantage of the combined use of mebeverine with the known therapeuticals is that the latter can be used in far lower doses, and hence result in less and less severe side-effects.

Said medicament for rectal administration may for example be a suppository, or an enema, preferably the latter.

The volume of the enema to be administered may vary. Larger volumes are required if higher parts of the colon are to be treated. The preferred volumes of the enema are between 20 and 100 ml. The concentration of mebeverine in the enema may vary between 1 and 20 g/l. The preferred concentration may vary be between 5 and 15 g/l. The carrier liquid in the enema generally is water, preferably saline.

According to the present invention use is made of mebeverine or a pharmaceutically acceptable acid addition salt thereof, such as a hydrochloride, palmoate or benzoate salt.

### EXAMPLE

Five patients with a history of ulcerative colitis treated with 5-ASA and/or corticisteroids, remained to have exacerbations under existing therapy.
All patients received mebeverine enema's in doses ranging from 250 mg in 25 ml saline to 500 mg in 50 ml saline.
In four patients addition of meberine enema to 5-ASA and corticoid therapy resulted in a pronounced improvement of colonic mucosa.
In two cases the total daily dose of corticosteroids could be lowered if mebeverine enema's were added.

## Claims

1. Use of mebeverine or a pharmaceutically acceptable acid addition salt thereof in the preparation of a medicament effective against non-specific inflammatory bowel disease.

2. Use according to claim 1, characterized in that said medicament is in a formulation suitable for rectal administration.

3. Use according to claim 1-2, characterized in that additionally one or more compounds effective in enhancement of rectal compliance and/or therapeutic efficacy are incorporated in said medicament.

4. Medicament for rectal administration characterized in that it contains mebeverine or a pharmaceutically acceptable acid addition salt thereof.

5. Medicament according to claim 4, characterized in that it additionally contains one or more compounds effective in enhancement of rectal compliance and/or therapeutic efficacy.

6. Medicament for oral administration characterized in that it contains mebeverine or a pharmaceutically acceptable acid addition salt thereof in a formulation that releases the mebeverine at the level of the diseased intestine.
